(19) Europäisches Patentamt
European Patent Office
Office européen des brevets

(11) **EP 4 131 150 A1**

(12) **EUROPEAN PATENT APPLICATION**
published in accordance with Art. 153(4) EPC

(43) Date of publication:
**08.02.2023 Bulletin 2023/06**

(51) International Patent Classification (IPC):
**G06T 7/00** (1995.01)

(21) Application number: **20928342.3**

(86) International application number:
**PCT/CN2020/116106**

(22) Date of filing: **18.09.2020**

(87) International publication number:
**WO 2021/196536 (07.10.2021 Gazette 2021/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.03.2020 CN 202010248890**

(71) Applicant: **Suzhou Rainmed Medical Technology Co., Ltd.**
**Suzhou, Jiangsu 215000 (CN)**

(72) Inventors:
• **WANG, Peng**
  **Suzhou, Jiangsu 215000 (CN)**
• **WANG, Zhiyuan**
  **Suzhou, Jiangsu 215000 (CN)**
• **CAO, Wenbin**
  **Suzhou, Jiangsu 215000 (CN)**
• **XU, Lei**
  **Suzhou, Jiangsu 215000 (CN)**
• **LIU, Guangzhi**
  **Suzhou, Jiangsu 215000 (CN)**

(74) Representative: **Arnold & Siedsma**
**Bezuidenhoutseweg 57**
**2594 AC The Hague (NL)**

(54) **METHOD AND APPARATUS FOR ACCURATELY EXTRACTING VESSEL CENTERLINE, ANALYSIS SYSTEM, AND STORAGE MEDIUM**

(57) The present application provides a method and apparatus for accurately extracting a vessel centerline, an analysis system, and a storage medium. The method for accurately extracting a vessel centerline comprises: selecting a coronary artery two-dimensional angiographic image in which a contrast medium is in a full state in a blood vessel; obtaining a blood vessel segment of interest from the coronary artery two-dimensional angiographic image; picking up a start point and an end point of the blood vessel segment of interest; obtaining local blood vessel region maps corresponding to the start point and the end point by segmentation from the coronary artery two-dimensional angiographic image; filtering the local blood vessel region maps to obtain a first image; performing vessel enhancement on the first image to obtain a second image; extracting an initial centerline of a blood vessel from the second image; and correcting the initial centerline of the blood vessel, and correcting points deviating from the center of the blood vessel to the center of the blood vessel to obtain an accurate centerline of the blood vessel. The present application achieves the accurate extraction of contour lines of a blood vessel, and the extraction is quick.

S100 — a frame of two-dimensional coronary angiography image when a blood vessel is fully filled with a contrast agent is selected

S200 — an interested vascular segment is acquired from the two-dimensional coronary angiography image

S300 — a starting point and an ending point of the interested vascular segment are picked

S400 — a local vascular area image corresponding to the starting point and the ending point is partitioned from the two-dimensional coronary angiography image

S500 — the local vascular area image is filtered to obtain a first image

S600 — a vascular enhancement is performed on the first image to obtain a second image

S700 — an initial vascular centerline is extracted from the second image

S800 — the initial vascular centerline is corrected, and a point deviating from a vascular center is corrected onto the vascular center to obtain an accurate vascular centerline

FIG. 1

EP 4 131 150 A1

**Description**

**TECHNICAL FIELD**

The present invention relates to the field of coronary artery medical technology, and particularly to a method and an apparatus for accurately extracting a vascular centerline, an analysis system and a storage medium.

**BACKGROUND**

The deposition of lipids and carbohydrates in human blood on the blood vessel wall may form plaques on the blood vessel wall, which then leads to vascular stenosis; especially the vascular stenosis near the coronary arteries of the heart may lead to insufficient blood supply to the heart muscle and induce coronary heart disease, angina pectoris and other diseases, which poses a serious risk to human health. According to statistics, there are about 11 million coronary heart disease patients in our country, and the number of patients treated with cardiovascular interventional surgery is increasing by more than 10% every year.

Although the conventional medical detection means such as coronary angiography (CAG), computed tomography (CT), etc., can display the severity of the coronary artery stenosis of the heart, the ischemia condition of the coronary artery cannot be accurately evaluated. In order to improve the accuracy of function evaluation of the coronary vessel, Pijls in 1993 proposed a new index for calculating the coronary vascular function through the pressure measurement - Fractional Flow Reserve (FFR). After long-term basic and clinical research, the FFR has become a golden standard for the function evaluation of the coronary stenosis.

The FFR usually refers to the fractional myocardial blood flow reserve, which is defined as the ratio of the maximum blood flow that the diseased coronary artery can provide to the maximum blood flow when the coronary artery is completely normal. Researches show that in the state of maximal coronary hyperemia, the ratio of blood flow can be replaced by a pressure value. That is, the measurement of the FFR value can be calculated by measuring the pressure at the distal stenosis of the coronary artery and the pressure at the proximal stenosis of the coronary artery through a pressure sensor under the state of maximum coronary hyperemia.

In the prior art, when blood vessel evaluation parameters are calculated through a three-dimensional model of the blood vessel, it is often necessary to extract the vascular centerline, but how to improve the accuracy of extracting the vascular centerline is always a problem that technicians need to solve.

**SUMMARY**

The present invention provides a method and an apparatus for accurately extracting a vascular centerline, a coronary artery analysis system, and a computer storage medium, to address the problem of how to accurately extract the vascular centerline.

In order to achieve the above purpose, in a first aspect, the present invention provides a method for accurately extracting a vascular centerline, including

selecting a frame of a two-dimensional coronary angiography image when a blood vessel is fully filled with a contrast agent;
acquiring an interested vascular segment from the two-dimensional coronary angiography image;
picking a starting point and an ending point of the interested vascular segment;
partitioning a local vascular area image corresponding to the starting point and the ending point from the two-dimensional coronary angiography image;
filtering the local vascular area image to obtain a first image;
performing a vascular enhancement on the first image to obtain a second image;
extracting an initial vascular centerline from the second image;
correcting the initial vascular centerline, and correcting a point deviating from a vascular center onto the vascular center to obtain an accurate vascular centerline.

Optionally, in the method for accurately extracting the vascular centerline, the partitioning the local vascular area image corresponding to the starting point and the ending point from the two-dimensional coronary angiography image includes:

picking at least one seed point of the interested vascular segment;
partitioning the second image according to an adjacent order of the starting point, the seed point and the ending point, to obtain at least two local vascular area images.

Optionally, in the method for accurately extracting the vascular centerline, the filtering the local vascular area image includes:

W and H representing a width and a height of a two-dimensional kernel function, respectively;

filtering the local vascular area image by the two-dimensional kernel function $g(x, y) = e^{-\frac{x^2+y^2}{2\sigma^2}}$;
wherein $x \in [0, W)$, $y \in [0,H)$; $\sigma$ represents a standard deviation, $\sigma = 0.5\text{-}5.0$, and $e$ represents a natural constant.

Optionally, in the method for accurately extracting the vascular centerline, $W=H=6\sigma+1$.

Optionally, in the method for accurately extracting the vascular centerline, the performing the vascular enhancement on the first image includes:

respectively calculating a plurality of matrix eigenvalues according to different standard deviations $\sigma$ and matrix sizes;
selecting a maximum value from all matrix eigenvalues as an output value, and an image corresponding to the output value being the second image after the vascular enhancement.

Optionally, in the method for accurately extracting the vascular centerline, the respectively calculating a plurality of matrix eigenvalues according to different standard deviations $\sigma$ and matrix sizes comprises:

acquiring a plurality of two-dimensional matrices $H(x, y)$ according to the different standard deviations $\sigma$ and matrix sizes;
acquiring a first parameter and a second parameter corresponding to each two-dimensional matrix;
acquiring matrix eigenvalues of each two-dimensional matrix according to the first parameter and the second parameter.

Optionally, in the method for accurately extracting the vascular centerline, the acquiring a plurality of two-dimensional matrices $H(x, y)$ according to the different standard deviations $\sigma$ and matrix sizes includes:

obtaining a secondary derivative of the image in an x-direction through a formula $I_{xx} = \dfrac{\partial^2 g(x, y)}{\partial^2 x} \otimes I(x, y)$ according to the two-dimensional kernel function $g(x, y)$;

obtaining a secondary derivative of the image in a y-direction through a formula $I_{yy} = \dfrac{\partial^2 g(x, y)}{\partial^2 y} \otimes I(x, y)$ according to the two-dimensional kernel function $g(x, y)$;
obtaining a secondary derivative of the image in a direction at an angle of 45 degrees to both the x-direction and the y-direction through a formula $I_{xy} = \dfrac{\partial^2 g(x, y)}{\partial x \partial y} \otimes I(x, y)$ according to the two-dimensional kernel function $g(x, y)$;

obtaining a two-dimensional matrix $H(x, y) = \begin{bmatrix} I_{xx} & I_{xy} \\ I_{xy} & I_{yy} \end{bmatrix}$ according to the $I_{xx}$, $I_{xy}$, and $I_{yy}$;
wherein $\sigma$ represents the standard deviation, $\sigma = 0.5\text{~}5.0$; $e$ represents a constant; $\otimes$ represents a convolution; $I(x, y)$ represents an image pixel value; $I_{xx}$, $I_{xy}$ and $I_{yy}$ respectively represent the secondary derivatives of the image in the x-direction, the direction at an angle of 45 degrees to both the x-direction and the y-direction, and the y-direction.

Optionally, in the method for accurately extracting the vascular centerline, the acquiring the first parameter and the second parameter corresponding to each two-dimensional matrix includes:

obtaining K by a formula $K = (I_{xx} + I_{yy})/2$ according to the $I_{xx}$ and $I_{yy}$;

obtaining Q by a formula $Q = \sqrt{I_{xx}I_{yy} - I_{xy}I_{xy}}$ according to the $I_{xx}$, $I_{xy}$ and $I_{yy}$;

obtaining the first parameter by a formula $\lambda_1 = K + \sqrt{K^2 - Q^2}$ according to the K and Q;

obtaining the second parameter by a formula $\lambda_2 = K - \sqrt{K^2 - Q^2}$ according to the $K$ and $Q$;
wherein $\lambda_1$ represents the first parameter; $\lambda_2$ represents the second parameter; the $I_{xx}$, $I_{xy}$ and $I_{yy}$ respectively represent the secondary derivatives of the image in the $x$-direction, the direction at an angle of 45 degrees to both the x-direction and the $y$-direction, and the $y$-direction.

Optionally, in the method for accurately extracting the vascular centerline, the acquiring the matrix eigenvalues of each two-dimensional matrix according to the first parameter and the second parameter includes:

obtaining $R_B$ by a formula $R_B = \dfrac{|\lambda_1|}{|\lambda_2|}$ according to the first parameter and the second parameter;

obtaining $S$ by a formula $S = \sqrt{\lambda_1^2 + \lambda_2^2}$ according to the first parameter and the second parameter;

obtaining a matrix eigenvalue $V$ by a formula
$$V = \begin{cases} 0 & if \quad \lambda_2 > 0 \\ \exp\left(-\dfrac{R_B^2}{2\beta^2}\right) \times \left(1 - \exp\left(-\dfrac{S^2}{2\gamma^2}\right)\right) \times |\lambda_2| & other \end{cases}$$
according to the $R_B$ and $S$, wherein $\beta$ represents a parameter configured to adjust a difference between linear and block, $\beta$=0.4~ 0.8; $\gamma$ represents a parameter configured to control an overall smoothness of a linear object, and $\gamma$ =3.0~5.0.

Optionally, in the method for accurately extracting the vascular centerline, the extracting an initial vascular centerline from the second image includes:

extracting a vascular skeleton from the second image;
picking a bifurcation point of the blood vessel, and creating an undirected image together with the bifurcation point, the starting point and the ending point;
searching for a shortest distance between two adjacent points in the undirected image in a direction of the vascular skeleton, to obtain all paths in a direction from the starting point to the ending point;
serving a vascular path in the all paths with the shortest distance in the direction from the starting point to the ending point of the undirected image as the initial vascular centerline.

Optionally, in the method for accurately extracting the vascular centerline, the correcting the initial vascular centerline and correcting the point deviating from the vascular center onto the vascular center to obtain the accurate vascular centerline comprises:

forming the initial vascular centerline by connecting a plurality of vascular center points, and applying a normal line to each vascular center point;
performing gray value statistics in the normal direction, if the image is the enhanced second image, moving the point deviating from the vascular center in the normal direction to a position with a maximum gray value in the vascular area, that is, completing the correction; if the image is the original image, moving the point deviating from the vascular center in the normal direction to a position with a minimum gray value in the vascular area, that is, completing the correction;
reconnecting the corrected vascular center points and obtaining the accurate vascular centerline.

In a second aspect, the present invention provides an apparatus for accurately extracting a vascular centerline, including: a two-dimensional coronary angiography image reading unit, an image selection unit, an interested vascular segment picking unit, an image partition unit, a filtering unit, an image enhancement unit, an initial centerline extraction unit, and a centerline correction unit which are connected in sequence;

the two-dimensional coronary angiography image reading unit is configured to read two-dimensional coronary angiography images obtained from at least two shooting angles at least one body position of a patient;
the image selection unit is configured to select a frame of two-dimensional coronary angiography image when a blood vessel is fully filled with a contrast agent from the two-dimensional coronary angiography image reading unit;
the interested vascular segment picking unit is configured to acquire an interested vascular segment from the two-dimensional coronary angiography image, and pick a starting point and an ending point of the interested vascular segment;

the image partition unit is configured to partition a local vascular area image corresponding to the starting point and the ending point from the two-dimensional coronary angiography image in the interested vascular segment picking unit;

the filtering unit is configured to filter the local vascular area image in the image partition unit to obtain a first image;

the image enhancement unit is configured to perform a vascular enhancement on the first image in the filtering unit to obtain a second image;

the initial centerline extraction unit is configured to extract the initial vascular centerline from the second image of the image enhancement unit;

the centerline correction unit is configured to correct the initial vascular centerline extracted by the initial centerline extraction unit, and correct a point deviating from a vascular center onto the vascular center to obtain an accurate vascular centerline.

Optionally, in the apparatus for accurately extracting the vascular centerline, the image enhancement unit includes: a two-dimensional matrix module, a parameter calculation module, a matrix eigenvalue calculation module, a matrix eigenvalue screening module, and a second image module which are connected in sequence;

the two-dimensional matrix module is configured to acquire a plurality of two-dimensional matrices according to different standard deviations and matrix sizes;

the parameter calculation module is configured to acquire a first parameter and a second parameter corresponding to each two-dimensional matrix according to the plurality of two-dimensional matrices in the two-dimensional matrix module;

the matrix eigenvalue module is configured to obtain matrix eigenvalues of each two-dimensional matrix according to the first parameter and the second parameter calculated by the parameter calculation module;

the matrix eigenvalue screening module is configured to select a maximum value from all matrix eigenvalues in the matrix eigenvalue module as an output value;

the second image module is configured to take an image corresponding to the output value as the second image after the vascular enhancement according to the output value of the matrix eigenvalue screening module.

In a third aspect, the present invention provides a coronary artery analysis system including the above-mentioned apparatus for accurately extracting the vascular centerline.

In a fourth aspect, the present invention provides a computer storage medium, when a computer program is executed by a processor, the method for accurately extracting the vascular centerline is implemented.

The beneficial effects brought by the solution provided in the embodiments of the present invention at least include:

The present invention provides a method for accurately extracting the vascular centerline. Due to the dark color of the contrast agent, if the contrast agent does not fully fill the blood vessels, the shape of the blood vessels may be incomplete, and the color of the edges is light, resulting in incomplete image of the blood vessels, which is easy to cause the problem of inaccurate picking of the blood vessel edge in the later stage. Accordingly, in the present invention, a frame of two-dimensional coronary angiography image when the blood vessel is fully filled with the contrast agent is selected, which can completely display the shape of the blood vessel.

In order to reduce the amount of computation, in the present invention the interested vascular segment is acquired from the two-dimensional coronary angiography image; the starting point and the ending point of the interested vascular segment are picked; the local vascular area image corresponding to the starting point and the ending point is partitioned from the two-dimensional coronary angiography image; since the local vascular area image is smaller than the coronary angiography image, so that the calculation amount is low, and the system response speed is fast.

Since there is noise in the image, in order to reduce the effect of the noise on the image, in the present invention, the local vascular area image is filtered to obtain the first image. In order to obtain clear blood vessels, in the present invention the vascular enhancement is performed on the first image to obtain the second image. In the present invention, the initial vascular centerline is extracted from the second image, and the speed is faster.

Since there are branches in the blood vessel, and the vascular centerline is related to the quality of the blood vessel and the edge of the blood vessel, there is an error in the points on the initial vascular centerline. In order to correct the initial vascular centerline, the gray value statistics is performed in the normal direction; if the image is the enhanced second image, the vascular center point deviating from the vascular center is moved in the normal direction to the position with the maximum gray value in the vascular area, that is, the correction is completed and the accurate vascular centerline is obtained, which improves the accuracy of the extraction of the vascular centerline.

## BRIEF DESCRIPTION OF THE DRAWINGS

The accompanying drawings described herein are utilized to provide further understanding of the present invention, and constitute a part of the present invention. The exemplary embodiments and the description thereof of the present invention are utilized to explain the present invention, rather than constituting an inappropriate limit to the present invention. In the drawings:

FIG. 1 is a flow chart showing a method for accurately extracting a vascular centerline according to the present invention;
FIG. 2 is a two-dimensional coronary angiography image according to the present invention;
FIG. 3 is a flow chart of a step S600 according to the present invention;
FIG. 4 is a flow chart of a step S610 according to the present invention;
FIG. 5 is a second image according to the present invention;
FIG. 6 is a flow chart of a step S700 according to the present invention;
FIG. 7 is a flow chart of a step S800 according to the present invention;
FIG. 8 is an image showing an extracted vascular centerline according to the present invention;
FIG. 9 is a structure block diagram illustrating an apparatus for accurately extracting a vascular centerline according to the present invention;
FIG. 10 is a structure block diagram illustrating an image enhancement unit 600 according to the present invention.

Reference signs are provided as follows:
100, two-dimensional coronary angiography image reading unit; 200, image selection unit; 300, interested vascular segment picking unit; 400, image partition unit; 500, filtering unit; 600, image enhancement unit; 610, two-dimensional matrix module; 620, parameter calculation module; 630, matrix eigenvalue calculation module; 640, matrix eigenvalue screening module; 650, second image module; 700, initial centerline extraction unit; 800, centerline correction unit.

## DETAILED DESCRIPTION

In order to make the purpose, technical solution and advantages of the present invention clearer, the technical solution of the present invention will be clearly and completely described below with reference to the specific embodiments and the corresponding drawings of the present invention. Obviously, the described embodiments are merely some embodiments, but not all embodiments of the present invention. Based on the embodiments of the present invention, all other embodiments obtained by those of ordinary skill in the art without creative efforts shall fall within the protection scope of the present invention.

Various embodiments of the present invention will be disclosed in the drawings below, and for the sake of clarity, many practical details will be provided together in the following description. However, it should be understood that these practical details should not be utilized to limit the present invention. That is, in some embodiments of the invention, these practical details are unnecessary. In addition, for the purpose of simplifying the drawings, some well-known structures and components will be shown in a simple schematic manner in the drawings.

In the prior art, when the vascular evaluation parameter is calculated through a three-dimensional model of a blood vessel, it is often necessary to extract a vascular contour. Since the blood vessel is curled and its edges are not clear, it is particularly difficult to extract the vascular contour, and the calculation data is huge and cumbersome, so that how to quickly extract the vascular contour and improve the accuracy of the extraction have always been problems that technicians need to solve.

Embodiment I

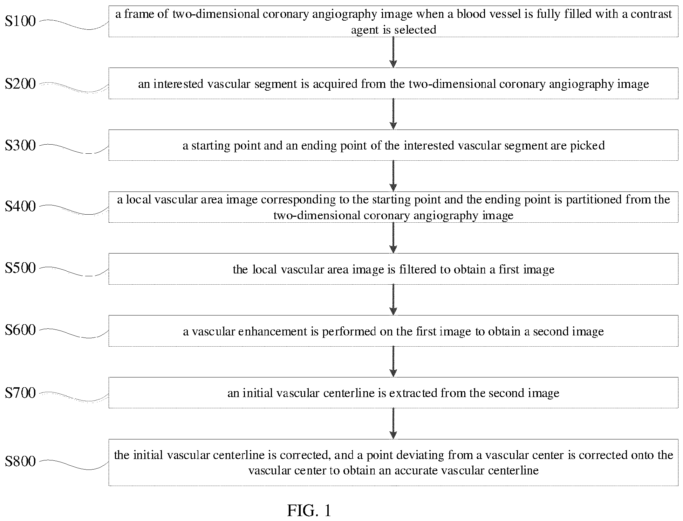

As shown in FIG. 1, in order to address the above problems, the present invention provides a method for accurately extracting a vascular centerline, including:

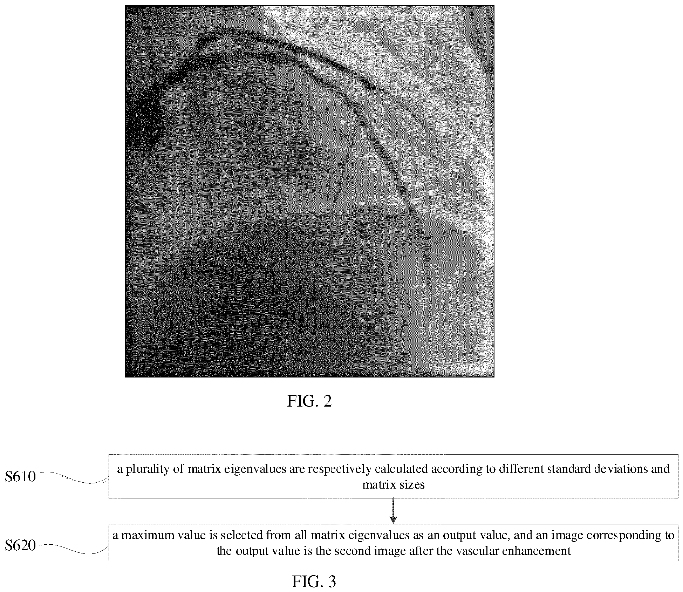

S100: a frame of two-dimensional coronary angiography image as shown in FIG. 2 when a blood vessel is fully filled with a contrast agent is selected.
S200: an interested vascular segment is acquired from the two-dimensional coronary angiography image.
S300: a starting point and an ending point of the interested vascular segment are picked.
S400: a local vascular area image corresponding to the starting point and the ending point is partitioned from the two-dimensional coronary angiography image.
S500: the local vascular area image is filtered to obtain a first image.
S600: a vascular enhancement is performed on the first image to obtain a second image.

S700: an initial vascular centerline is extracted from the second image.

S800: the initial vascular centerline is corrected, and a point deviating from a vascular center is corrected onto the vascular center to obtain an accurate vascular centerline.

The present invention provides a method for accurately extracting the vascular centerline. Due to the dark color of the contrast agent, if the contrast agent does not fully fill the blood vessel, the shape of the blood vessel is incomplete, and the color of the edges is light, which results in incomplete image of the blood vessel, and is easy to cause inaccurate pickup of the edges of the blood vessel in the later stage. Therefore, in the present invention, a frame of two-dimensional coronary angiography image when the blood vessel is fully filled with the contrast agent is selected, accordingly the shape of the blood vessel can be displayed completely.

In order to reduce the amount of computation, in the present invention, the interested vascular segment is acquired from the two-dimensional coronary angiography image; the starting point and ending point of the interested vascular segment are selected; the local vascular area image corresponding to the starting point and the ending point is partitioned from the two-dimensional coronary angiography image, since the local vascular area image is smaller than the coronary angiography image, the amount of the calculation is low and the system response speed is fast.

Since there is a noise in the image, in order to reduce the effect of the noise to the image, in the present invention the local vascular area image is filtered to obtain the first image. In order to obtain the clear blood vessel, in the present invention the vascular enhancement is performed on the first image to obtain the second image. In the present invention, the initial vascular centerline is extracted from the second image, accordingly, the speed is faster.

Since the blood vessel has branches, and the centerline of the blood vessel is related to the quality of the blood vessel and the edges of the blood vessel, there is an error in a point on the initial vascular centerline. In order to correct the initial vascular centerline, in the present invention, gray value statistics are performed in a normal direction of the initial vascular centerline. If the image is the enhanced second image, the point deviating from the vascular center is moved in the normal direction to a position with the maximum gray value in the vascular area, that is, the correction is completed. Accordingly, the accurate vascular centerline is obtained, thereby improving the accuracy of the extraction of the vascular centerline.

Embodiment II

As shown in FIG. 1, in order to address the above problem, the present invention provides a method for accurately extracting a vascular centerline, which includes the following steps.

S100: a frame of a two-dimensional coronary angiography image as shown in FIG. 2 when a blood vessel is fully filled with a contrast agent is selected.

S200: an interested vascular segment is acquired from the two-dimensional coronary angiography image.

S300: a starting point and an ending point of the interested vascular segment are picked.

S400: a local vascular area image corresponding to the starting point and the ending point is partitioned from the two-dimensional coronary angiography image. Preferably, in order to improve the accuracy of picking of the blood vessel, in the present invention, at least one seed point of the interested vascular segment between the starting point and the ending point can be additionally picked; the second image is partitioned according to an adjacent order of the starting point, the seed point and the ending point, to obtain at least two local vascular area images.

S500: the local vascular area image is filtered to obtain a first image, which includes:

$W$ and $H$ represent a width and a height of a two-dimensional kernel function, respectively;

the local vascular area image is filtered by two-dimensional kernel function $g(x, y) = e^{-\frac{x^2+y^2}{2\sigma^2}}$ ;

where $x \in [0, W)$, $y \in [0, H)$; $\sigma$ represents a standard deviation, $\sigma = 0.5\text{-}5.0$, and $e$ represents a natural constant.

Preferably, in order to reduce the amount of computation, $\sigma$ can be valuated within an interval of $\sigma = \{0.5, 1.0, 1.5, 2.0, 2.5, 3.0, 3.5, 4.0, 4.5, 5.0\}$, and the calculation is performed when $W = H = 6\sigma + 1$, thereby improving the computing speed.

S600: a vascular enhancement is performed on the first image to obtain a second image, shown in FIG. 3, which includes:

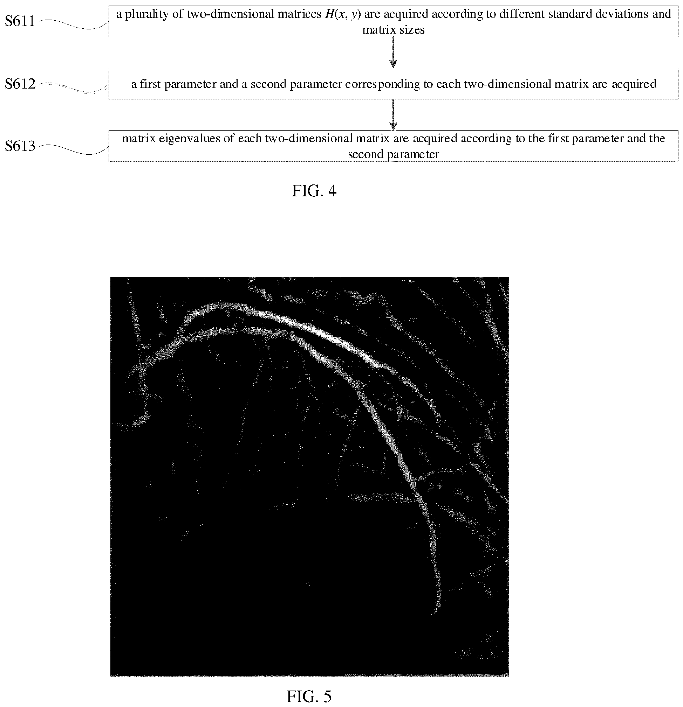

S610: a plurality of matrix eigenvalues are respectively calculated according to different standard deviations $\sigma$ and matrix sizes, as shown in FIG. 4, which includes following steps.

S611: a plurality of two-dimensional matrices $H(x, y)$ are acquired according to different standard deviations $\sigma$ and matrix sizes, specifically,

A) a secondary derivative of the image in the *x*-direction is obtained through the formula

$$I_{xx} = \frac{\partial^2 g(x, y)}{\partial^2 x} \otimes I(x, y)$$

according to the two-dimensional kernel function *g(x, y)*;

B) a secondary derivative of the image in the *y*-direction is obtained through the formula

$$I_{yy} = \frac{\partial^2 g(x, y)}{\partial^2 y} \otimes I(x, y)$$

according to the two-dimensional kernel function *g(x, y)*;

C) a secondary derivative of the image in a direction at an angle of 45 degrees to both the x-direction and the *y*-direction is obtained through the formula 

$$I_{xy} = \frac{\partial^2 g(x, y)}{\partial x \partial y} \otimes I(x, y)$$

according to the two-dimensional kernel function *g(x, y)*;

D) a two-dimensional matrix 

$$H(x, y) = \begin{bmatrix} I_{xx} & I_{xy} \\ I_{xy} & I_{yy} \end{bmatrix}$$

is obtained according to $I_{xx}$, $I_{xy}$, and $I_{yy}$;

where $\sigma$ represents the standard deviation, $\sigma = 0.5\text{-}5.0$; e represents a constant; $\otimes$ represents a convolution, $I(x, y)$ represents an image pixel value; $I_{xx}$, $I_{xy}$ and $I_{yy}$ respectively represent the secondary derivatives of the image in the x-direction, the direction at an angle of 45 degrees to both the x-direction and the *y*-direction, and the *y*-direction.
S612: a first parameter and a second parameter corresponding to each two-dimensional matrix are acquired, specifically,

I) *K* is obtained by a formula $K = (I_{xx} + I_{vv}) / 2$ according to the $I_{xx}$ and $I_{yy}$;

II) *Q* is obtained by a formula $Q = \sqrt{I_{xx}I_{yy} - I_{xy}I_{xy}}$ according to $I_{xx}$, $I_{xy}$ and $I_{yy}$;

III) the first parameter is obtained by a formula $\lambda_1 = K + \sqrt{K^2 - Q^2}$ according to the *K* and *Q*;

IV) the second parameter is obtained by a formula $\lambda_2 = K - \sqrt{K^2 - Q^2}$ according to the *K* and *Q*;

where, $\lambda_1$ represents the first parameter; $\lambda_2$ represents the second parameter; $I_{xx}$, $I_{xy}$ and $I_{yy}$ respectively represent the secondary derivatives of the image in the *x*-direction, the direction at an angle of 45 degrees to both the *x*-direction and the *y*-direction, and the *y*-direction.
S613: matrix eigenvalues of each two-dimensional matrix are acquired according to the first parameter and the second parameter, specifically,

① $R_B$ is obtained by a formula $R_B = \frac{|\lambda_1|}{|\lambda_2|}$ according to the first parameter and the second parameter;

② S is obtained by a formula $S = \sqrt{\lambda_1^2 + \lambda_2^2}$ according to the first parameter and the second parameter;

③ the matrix eigenvalue *V* is obtained by a formula 

$$V = \begin{cases} 0 & if \quad \lambda_2 > 0 \\ \exp\left(-\frac{R_B^2}{2\beta^2}\right) \times \left(1 - \exp\left(-\frac{S^2}{2\gamma^2}\right)\right) \times |\lambda_2| & other \end{cases}$$

according to the $R_B$ and *S*; where $\beta$ represents a parameter configured to adjust a difference between linear and block; $\beta = 0.4 \sim 0.8$; $\gamma$ represents a parameter configured to control an overall smoothness of a linear object, and $\gamma = 3.0 \sim 5.0$.

In the present invention, different standard deviations are utilized to obtain different *W* and *H,* in order to enhance blood vessels with different diameters; through the calculation of the image enhancement, even for the blood vessels with unclear images, the relatively clear enhanced images of the blood vessels can also be obtained by the present invention, which has wide applicability.

S620: a maximum value is selected from all matrix eigenvalues as an output value, and an image corresponding to the output value is the second image after the vascular enhancement as shown in FIG. 5.

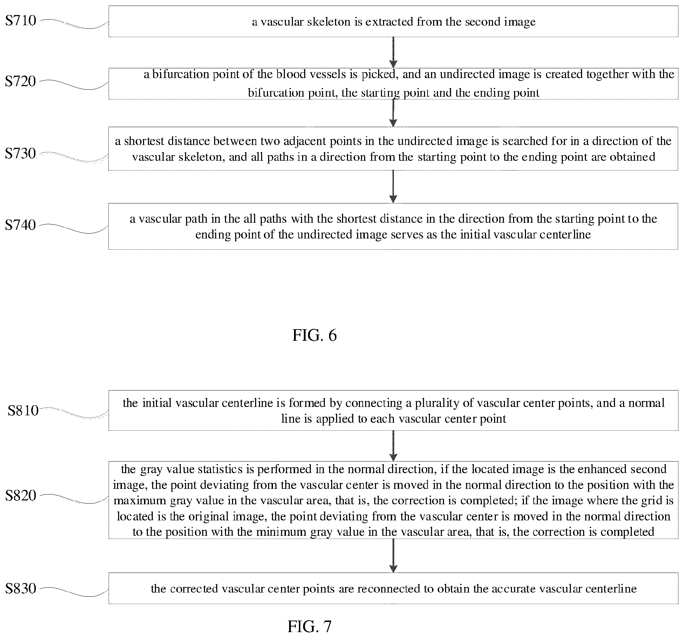

S700: an initial vascular centerline is extracted from the second image, as shown in FIG. 6, which includes:

S710: a vascular skeleton is extracted from the second image;

S720: a bifurcation point of the blood vessels is picked, and an undirected image is created together with the bifurcation point, the starting point and the ending point;

S730: a shortest distance between two adjacent points in the undirected image is searched for in a direction of the vascular skeleton, and all paths in a direction from the starting point to the ending point are obtained;

S740: a vascular path in the all paths with the shortest distance in the direction from the starting point to the ending point of the undirected image serves as the initial vascular centerline.

S800: the initial vascular centerline is corrected, and a point deviating from a vascular center is corrected onto the vascular center to obtain an accurate vascular centerline, as shown in FIG. 7, specifically,

S810: the initial vascular centerline is formed by connecting a plurality of vascular center points, and a normal line is applied to each vascular center point;

S820: the gray value statistics is performed in the normal direction, if the located image is the enhanced second image, the point deviating from the vascular center is moved in the normal direction to the position with the maximum gray value in the vascular area, that is, the correction is completed; if the image where the grid is located is the original image, the point deviating from the vascular center is moved in the normal direction to the position with the minimum gray value in the vascular area, that is, the correction is completed;

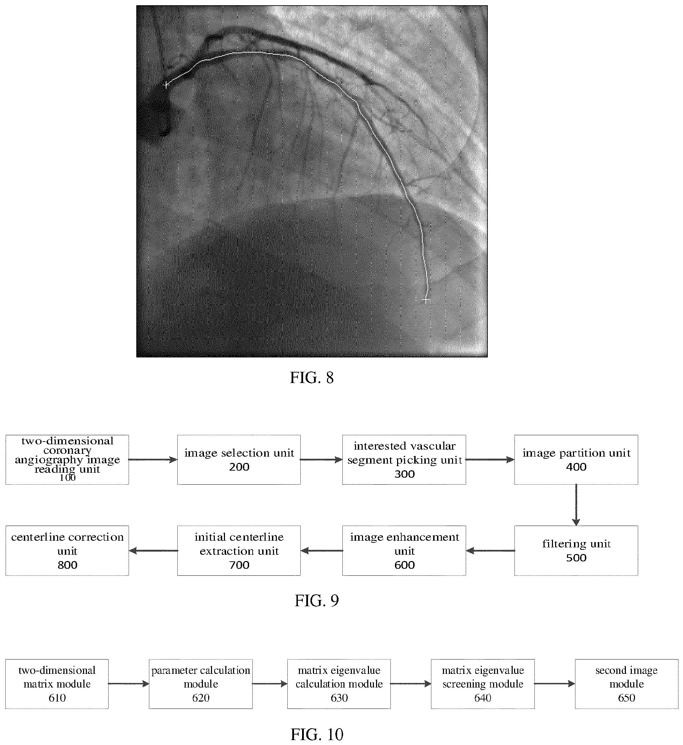

S830: the corrected vascular center points are reconnected to obtain the accurate vascular centerline as shown in FIG. 8.

In a second aspect, the present invention provides an apparatus for accurately extracting a vascular centerline, as shown in FIG. 9, including: a two-dimensional coronary angiography image reading unit 100, an image selection unit 200, an interested vascular segment picking unit 300, an image partition unit 400, a filtering unit 500, an image enhancement unit 600, an initial centerline extraction unit 700, and a centerline correction unit 800, which are connected in sequence. The two-dimensional coronary angiography image reading unit 100 is configured to read two-dimensional coronary angiography images obtained from at least two shooting angles at least one body position of a patient. The image selection unit 200 is configured to select a frame of two-dimensional coronary angiography image when a blood vessel is fully filled with a contrast agent from the two-dimensional coronary angiography image reading unit. The interested vascular segment picking unit 300 is configured to acquire an interested vascular segment from the two-dimensional coronary angiography image, and pick a starting point and an ending point of the interested vascular segment. The image partition unit 400 is configured to partition the local vascular area image corresponding to the starting point and the ending point from the two-dimensional coronary angiography image in the interested vascular segment picking unit. The filtering unit 500 is configured to filter the local vascular area image in the image partition unit to obtain a first image. The image enhancement unit 600 is configured to perform a vascular enhancement on the first image in the filtering unit to obtain a second image. The initial centerline extraction unit 700 is configured to extract the initial vascular centerline from the second image of the image enhancement unit. The centerline correction unit 800 is configured to correct the initial vascular centerline extracted by the initial centerline extraction unit, and correct a point deviating from a vascular center onto the vascular center to obtain an accurate vascular centerline.

Optionally, in the above-mentioned apparatus for accurately extracting the vascular centerline, as shown in FIG. 10, the image enhancement unit 600 includes: a two-dimensional matrix module 610, a parameter calculation module 620, a matrix eigenvalue calculation module 630, a matrix eigenvalue screening module 640, and a second image module 650 which are connected in sequence. The two-dimensional matrix module 610 is configured to acquire a plurality of two-dimensional matrices according to different standard deviations and matrix sizes. The parameter calculation module 620 is configured to acquire a first parameter and a second parameter corresponding to each two-dimensional matrix according to the plurality of two-dimensional matrices in the two-dimensional matrix module. The matrix eigenvalue module 630 is configured to obtain matrix eigenvalues of each two-dimensional matrix according to the first parameter and the second parameter calculated by the parameter calculation module 620. The matrix eigenvalue screening module 640 is configured to select a maximum value from all matrix eigenvalues in the matrix eigenvalue module 630 as an output value. The second image module 650 is configured to take an image corresponding to the output value as the second image after the vascular enhancement according to the output value of the matrix eigenvalue screening module 640.

In a third aspect, the present invention provides a coronary artery analysis system, including the above-mentioned apparatus for accurately extracting the vascular centerline.

In a fourth aspect, the present invention provides a computer storage medium, and when the computer program is executed by a processor, the above-mentioned method for accurately extracting the vascular centerline is implemented.

As will be appreciated by one skilled in the art, various aspects of the present invention may be implemented as a system, a method or a computer program product. Accordingly, various aspects of the present invention may be implemented in the form of an entirely hardware implementation, an entirely software implementation (including firmware, resident software, microcode, etc.), or a combination of hardware and software aspects, which may be collectively referred to herein as a "circuit", "module", or "system". Furthermore, in some embodiments, various aspects of the present invention may also be implemented in the form of a computer program product on one or more computer readable media including computer readable program codes. Implementation of the method and/or system of embodiments of the present invention may involve performing or completing selected tasks manually, automatically, or a combination thereof.

For example, hardware for performing selected tasks according to embodiments of the present invention may be implemented as a chip or a circuit. As software, the selected tasks according to embodiments of the present invention may be implemented as a plurality of software instructions executed by a computer using any appropriate operating system. In an exemplary embodiment of the present invention, one or more tasks according to exemplary embodiments of a method and/or system as herein are performed by a data processor, such as a computing platform for executing a plurality of instructions. Optionally, the data processor includes a transitory memory for storing instructions and/or data and/or non-transitory memory for storing instructions and/or data, such as a magnetic hard disk and/or movable media. Optionally, a network connection is further provided. Optionally, a displayer and/or user input device is further provided, such as a keyboard or mouse.

Any combination of one or more computer-readable media may be utilized. The computer-readable medium may be a computer-readable signal medium or a computer-readable storage medium. The computer-readable storage medium can be, for example, but not limited to, an electrical, magnetic, optical, electromagnetic, infrared, or semiconductor system, apparatus or device, or a combination of any of the above. More specific examples (non-exhaustive list) of the computer-readable storage medium may include the following.

Electrically connected and portable computer disk, hard disk, random access memory (RAM), read only memory (ROM), erasable programmable read only memory (EPROM or flash memory), optical fiber, portable compact disk read only memory (CD-ROM), optical storage device, magnetic storage device having one or more wires, or any appropriate combination of the above. In the present invention, the computer-readable storage medium can be any tangible medium that contains or stores a program that can be used by or in conjunction with an instruction execution system, apparatus, or device.

The computer-readable signal medium may include a propagated data signal in a baseband or as part of a carrier wave, carrying computer-readable program codes. Such propagated data signal may take a variety of forms, including but not limited to an electromagnetic signal, an optical signal, or any appropriate combination of the foregoing. A computer-readable signal medium can also be any computer-readable medium other than a computer-readable storage medium that can transmit, propagate, or transport the program used by or in connection with the instruction execution system, apparatus, or device.

The program codes included in the computer-readable medium may be transmitted using any appropriate medium, including but not limited to wireless, wired, optical fiber cable, RF, etc., or any appropriate combination of the foregoing.

For example, the computer program codes for performing operations of various aspects of the present invention may be written in any combination of one or more programming languages, including object-oriented programming languages such as Java, Smalltalk, C++, and conventional procedural programming languages, such as "C" programming language or similar programming languages. The program codes may be executed entirely on the user's computer, partly on the user's computer, independently as a software package, partly on the user's computer and partly on a remote computer, or entirely on a remote computer or a server. In the case of a remote computer, the remote computer can be connected to the user's computer through any kind of network including a local area network (LAN) or a wide area network (WAN), or can be connected to an external computer (e.g., connected through the Internet by means of an Internet service provider).

It should be appreciated that each block of the flowcharts and/or block diagrams, and combinations of blocks in the flowcharts and/or block diagrams can be implemented by computer program instructions. These computer program instructions may be provided to a processor of a general-purpose computer, a special-purpose computer or other programmable data processing devices to produce a machine such that when the computer program instructions are executed by the processor of the computer or other programmable data processing devices, a device which can implement the functions/acts specified in one or more blocks in the flowcharts and/or block diagrams is produced.

These computer program instructions can also be stored in a computer-readable medium, and the instructions cause a computer, other programmable data processing devices, or other devices to operate in a particular manner, thereby the instructions stored in the computer-readable medium produce an article of manufacture including instructions for implementing the functions/acts specified in one or more blocks of the flowcharts and/or block diagrams.

The computer program instructions can also be loaded onto a computer (e.g., a coronary artery analysis system) or other programmable data processing devices to execute a series of operational steps on the computer, other programmable data processing devices or other devices to produce a computer-implemented process, such that the instructions executed on the computer, other programmable devices, or other devices provide a process for implementing the functions/acts specified in the flowcharts and/or one or more block diagrams.

The above specific embodiments of the present invention further detail the purpose, technical solution and beneficial effects of the present invention, it should be appreciated that the above are merely specific embodiments of the present invention, and are not intended to limit the present invention. Any modification, equivalent replacement, improvement, etc., made within the spirit and principle of the present invention shall all fall within the protection scope of the present invention.

**Claims**

1. A method for accurately extracting a vascular centerline, comprising:

   selecting a frame of a two-dimensional coronary angiography image when a blood vessel is fully filled with a contrast agent;
   acquiring an interested vascular segment from the two-dimensional coronary angiography image;
   picking a starting point and an ending point of the interested vascular segment;
   partitioning a local vascular area image corresponding to the starting point and the ending point from the two-dimensional coronary angiography image;
   filtering the local vascular area image to obtain a first image;
   performing a vascular enhancement on the first image to obtain a second image;
   extracting an initial vascular centerline from the second image;
   correcting the initial vascular centerline, and correcting a point deviating from a vascular center onto the vascular center to obtain an accurate vascular centerline.

2. The method for accurately extracting the vascular centerline according to claim 1, wherein the partitioning the local vascular area image corresponding to the starting point and the ending point from the two-dimensional coronary angiography image comprises:

   picking at least one seed point of the interested vascular segment;
   partitioning the second image according to an adjacent order of the starting point, the seed point and the ending point, to obtain at least two local vascular area images.

3. The method for accurately extracting the vascular centerline according to claim 1, wherein the filtering the local vascular area image comprises:

   W and H representing a width and a height of a two-dimensional kernel function, respectively;

   filtering the local vascular area image by the two-dimensional kernel function $g(x, y) = e^{-\frac{x^2 + y^2}{2\sigma^2}}$ ;
   wherein $x \in [0, W)$, $y \in [0, H)$; $\sigma$ represents a standard deviation, $\sigma = 0.5\text{-}5.0$, and $e$ represents a natural constant.

4. The method for accurately extracting the vascular centerline according to claim 3, wherein $W=H=6\sigma+1$.

5. The method for accurately extracting the vascular centerline according to claim 3 or 4, wherein the performing the vascular enhancement on the first image comprises:

   respectively calculating a plurality of matrix eigenvalues according to different standard deviations $\sigma$ and matrix sizes;
   selecting a maximum value from all matrix eigenvalues as an output value, and an image corresponding to the output value being the second image after the vascular enhancement.

6. The method for accurately extracting the vascular centerline according to claim 5, wherein the respectively calculating the plurality of matrix eigenvalues according to different standard deviations $\sigma$ and matrix sizes comprises:

   acquiring a plurality of two-dimensional matrices $H(x, y)$ according to the different standard deviations $\sigma$ and

matrix sizes;

acquiring a first parameter and a second parameter corresponding to each two-dimensional matrix;

acquiring matrix eigenvalues of each two-dimensional matrix according to the first parameter and the second parameter.

7. The method for accurately extracting the vascular centerline according to claim 6, wherein the acquiring the plurality of two-dimensional matrices $H(x, y)$ according to the different standard deviations σ and matrix sizes comprises:

obtaining a secondary derivative of the image in an $x$-direction through a formula

$$I_{xx} = \frac{\partial^2 g(x, y)}{\partial^2 x} \otimes I(x, y)$$

according to the two-dimensional kernel function $g(x, y)$;

obtaining a secondary derivative of the image in a $y$-direction through a formula

$$I_{yy} = \frac{\partial^2 g(x, y)}{\partial^2 y} \otimes I(x, y)$$

according to the two-dimensional kernel function $g(x, y)$;

obtaining a secondary derivative of the image in a direction at an angle of 45 degrees to both the x-direction and the $y$-direction through a formula

$$I_{xy} = \frac{\partial^2 g(x, y)}{\partial x \partial y} \otimes I(x, y)$$

according to the two-dimensional kernel function $g(x, y)$;

obtaining a two-dimensional matrix

$$H(x, y) = \begin{bmatrix} I_{xx} & I_{xy} \\ I_{xy} & I_{yy} \end{bmatrix}$$

according to the $I_{xx}$, $I_{xy}$, and $I_{yy}$;

wherein σ represents the standard deviation, σ =0.5~5.0; $e$ represents a constant; $\otimes$ represents a convolution; $I(x, y)$ represents an image pixel value; $I_{xx}$, $I_{xy}$ and $I_{yy}$ respectively represent the secondary derivatives of the image in the $x$-direction, the direction at an angle of 45 degrees to both the $x$-direction and the $y$-direction, and the $y$-direction.

8. The method for accurately extracting the vascular centerline according to claim 7, wherein the acquiring the first parameter and the second parameter corresponding to each two-dimensional matrix comprises:

obtaining $K$ by a formula $K = (I_{xx} + I_{yy}) / 2$ according to the $I_{xx}$ and $I_{yy}$;

obtaining $Q$ by a formula $Q = \sqrt{I_{xx}I_{yy} - I_{xy}I_{xy}}$ according to the $I_{xx}$, $I_{xy}$ and $I_{yy}$;

obtaining the first parameter by a formula $\lambda_1 = K + \sqrt{K^2 - Q^2}$ according to the $K$ and $Q$;

obtaining the second parameter by a formula $\lambda_2 = K - \sqrt{K^2 - Q^2}$ according to the $K$ and Q;

wherein $\lambda_1$ represents the first parameter; $\lambda_2$ represents the second parameter; the $I_{xx}$, $I_{xy}$ and $I_{yy}$ respectively represent the secondary derivatives of the image in the x-direction, the direction at an angle of 45 degrees to both the x-direction and the $y$-direction, and the $y$-direction.

9. The method for accurately extracting the vascular centerline according to claim 7, wherein the acquiring the matrix eigenvalues of each two-dimensional matrix according to the first parameter and the second parameter comprises:

obtaining $R_B$ by a formula $R_B = \frac{|\lambda_1|}{|\lambda_2|}$ $R_B$ according to the first parameter and the second parameter;

obtaining $S$ by a formula $S = \sqrt{\lambda_1^2 + \lambda_2^2}$ according to the first parameter and the second parameter;

obtaining a matrix eigenvalue $V$ by a formula

$$V = \begin{cases} 0 & if \quad \lambda_2 > 0 \\ \exp\left(-\frac{R_B^2}{2\beta^2}\right) \times \left(1 - \exp\left(-\frac{S^2}{2\gamma^2}\right)\right) \times |\lambda_2| & other \end{cases}$$

according to the $R_B$ and $S$, wherein β represents a parameter configured to adjust a difference between linear and block, β=0.4~ 0.8; γ represents a parameter configured to control an overall smoothness of a linear object, and γ =3.0~5.0.

10. The method for accurately extracting the vascular centerline according to claim 1, wherein the extracting an initial vascular centerline from the second image comprises:

> extracting a vascular skeleton from the second image;
> picking a bifurcation point of the blood vessel, and creating an undirected image together with the bifurcation point, the starting point and the ending point;
> searching for a shortest distance between two adjacent points in the undirected image in a direction of the vascular skeleton, to obtain all paths in a direction from the starting point to the ending point;
> serving a vascular path in the all paths with the shortest distance in the direction from the starting point to the ending point of the undirected image as the initial vascular centerline.

11. The method for accurately extracting the vascular centerline according to claim 10, wherein the correcting the initial vascular centerline and correcting the point deviating from the vascular center onto the vascular center to obtain the accurate vascular centerline comprises:

> forming the initial vascular centerline by connecting a plurality of vascular center points, and applying a normal line to each vascular center point;
> performing gray value statistics in the normal direction, if the image is the enhanced second image, moving the point deviating from the vascular center in the normal direction to a position with a maximum gray value in the vascular area, that is, completing the correction; if the image is the original image, moving the point deviating from the vascular center in the normal direction to a position with a minimum gray value in the vascular area, that is, completing the correction;
> reconnecting the corrected vascular center points and obtaining the accurate vascular centerline.

12. An apparatus for accurately extracting a vascular centerline, applied to the method for accurately extracting the vascular centerline of any one of claims 1 to 11, comprising:

> a two-dimensional coronary angiography image reading unit, an image selection unit, an interested vascular segment picking unit, an image partition unit, a filtering unit, an image enhancement unit, an initial centerline extraction unit, and a centerline correction unit which are connected in sequence; wherein
> the two-dimensional coronary angiography image reading unit is configured to read two-dimensional coronary angiography images obtained from at least two shooting angles at least one body position of a patient;
> the image selection unit is configured to select a frame of two-dimensional coronary angiography image when a blood vessel is fully filled with a contrast agent from the two-dimensional coronary angiography image reading unit;
> the interested vascular segment picking unit is configured to acquire an interested vascular segment from the two-dimensional coronary angiography image, and pick a starting point and an ending point of the interested vascular segment;
> the image partition unit is configured to partition a local vascular area image corresponding to the starting point and the ending point from the two-dimensional coronary angiography image in the interested vascular segment picking unit;
> the filtering unit is configured to filter the local vascular area image in the image partition unit to obtain a first image;
> the image enhancement unit is configured to perform a vascular enhancement on the first image in the filtering unit to obtain a second image;
> the initial centerline extraction unit is configured to extract the initial vascular centerline from the second image of the image enhancement unit;
> the centerline correction unit is configured to correct the initial vascular centerline extracted by the initial centerline extraction unit, and correct a point deviating from a vascular center onto the vascular center to obtain an accurate vascular centerline.

13. A coronary artery analysis system, comprising the apparatus for accurately extracting the vascular centerline of claim 12.

14. A computer storage medium, wherein when a computer program is executed by a processor, the method for accurately extracting the vascular centerline of any one of claims 1 to 11 is implemented.

S100 — a frame of two-dimensional coronary angiography image when a blood vessel is fully filled with a contrast agent is selected

S200 — an interested vascular segment is acquired from the two-dimensional coronary angiography image

S300 — a starting point and an ending point of the interested vascular segment are picked

S400 — a local vascular area image corresponding to the starting point and the ending point is partitioned from the two-dimensional coronary angiography image

S500 — the local vascular area image is filtered to obtain a first image

S600 — a vascular enhancement is performed on the first image to obtain a second image

S700 — an initial vascular centerline is extracted from the second image

S800 — the initial vascular centerline is corrected, and a point deviating from a vascular center is corrected onto the vascular center to obtain an accurate vascular centerline

FIG. 1

FIG. 2

S610 — a plurality of matrix eigenvalues are respectively calculated according to different standard deviations and matrix sizes

S620 — a maximum value is selected from all matrix eigenvalues as an output value, and an image corresponding to the output value is the second image after the vascular enhancement

FIG. 3

S611 — | a plurality of two-dimensional matrices $H(x, y)$ are acquired according to different standard deviations and matrix sizes |

S612 — | a first parameter and a second parameter corresponding to each two-dimensional matrix are acquired |

S613 — | matrix eigenvalues of each two-dimensional matrix are acquired according to the first parameter and the second parameter |

FIG. 4

FIG. 5

S710 — a vascular skeleton is extracted from the second image

S720 — a bifurcation point of the blood vessels is picked, and an undirected image is created together with the bifurcation point, the starting point and the ending point

S730 — a shortest distance between two adjacent points in the undirected image is searched for in a direction of the vascular skeleton, and all paths in a direction from the starting point to the ending point are obtained

S740 — a vascular path in the all paths with the shortest distance in the direction from the starting point to the ending point of the undirected image serves as the initial vascular centerline

FIG. 6

S810 — the initial vascular centerline is formed by connecting a plurality of vascular center points, and a normal line is applied to each vascular center point

S820 — the gray value statistics is performed in the normal direction, if the located image is the enhanced second image, the point deviating from the vascular center is moved in the normal direction to the position with the maximum gray value in the vascular area, that is, the correction is completed; if the image where the grid is located is the original image, the point deviating from the vascular center is moved in the normal direction to the position with the minimum gray value in the vascular area, that is, the correction is completed

S830 — the corrected vascular center points are reconnected to obtain the accurate vascular centerline

FIG. 7

FIG. 8

| two-dimensional coronary angiography image reading unit 100 | → | image selection unit 200 | → | interested vascular segment picking unit 300 | → | image partition unit 400 |

| centerline correction unit 800 | ← | initial centerline extraction unit 700 | ← | image enhancement unit 600 | ← | filtering unit 500 |

FIG. 9

| two-dimensional matrix module 610 | → | parameter calculation module 620 | → | matrix eigenvalue calculation module 630 | → | matrix eigenvalue screening module 640 | → | second image module 650 |

FIG. 10

## INTERNATIONAL SEARCH REPORT

| International application No. |
| --- |
| **PCT/CN2020/116106** |

**A. CLASSIFICATION OF SUBJECT MATTER**

G06T 7/00(2017.01)i

According to International Patent Classification (IPC) or to both national classification and IPC

**B. FIELDS SEARCHED**

Minimum documentation searched (classification system followed by classification symbols)

G06T

Documentation searched other than minimum documentation to the extent that such documents are included in the fields searched

Electronic data base consulted during the international search (name of data base and, where practicable, search terms used)

DWPI, SIPOABS, CNABS, CNKI: 图像, 血管, 兴趣, 区域, 滤波, 中心线, 起点, 终点, 增强, 校正, image, blood vessel, interest, region, filter, line, point, enhance, adjust

**C. DOCUMENTS CONSIDERED TO BE RELEVANT**

| Category* | Citation of document, with indication, where appropriate, of the relevant passages | Relevant to claim No. |
| --- | --- | --- |
| X | CN 101393644 A (HUAZHONG UNIVERSITY OF SCIENCE AND TECHNOLOGY) 25 March 2009 (2009-03-25) <br> description, page 2 line 13 - page 9 line 22 | 1-14 |
| A | CN 101283911 A (NORTH CHINA ELECTRIC POWER UNIVERSITY) 15 October 2008 (2008-10-15) <br> entire document | 1-14 |
| A | CN 101425186 A (HUAZHONG UNIVERSITY OF SCIENCE AND TECHNOLOGY) 06 May 2009 (2009-05-06) <br> entire document | 1-14 |
| A | US 2019053780 A1 (MAYO FOUND MEDICAL EDUCATION & RES) 21 February 2019 (2019-02-21) <br> entire document | 1-14 |
| A | US 2018109698 A1 (IMAGO SYSTEMS INC) 19 April 2018 (2018-04-19) <br> entire document | 1-14 |

☐ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **16 December 2020** | **07 January 2021** |

| Name and mailing address of the ISA/CN | Authorized officer |
| --- | --- |
| **China National Intellectual Property Administration (ISA/CN)** <br> **No. 6, Xitucheng Road, Jimenqiao, Haidian District, Beijing 100088** <br> **China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.

**PCT/CN2020/116106**

| Patent document cited in search report | | | Publication date (day/month/year) | Patent family member(s) | | | Publication date (day/month/year) |
|---|---|---|---|---|---|---|---|
| CN | 101393644 | A | 25 March 2009 | CN | 101393644 | B | 04 August 2010 |
| CN | 101283911 | A | 15 October 2008 | CN | 101283911 | B | 25 August 2010 |
| CN | 101425186 | A | 06 May 2009 | CN | 101425186 | B | 28 March 2012 |
| US | 2019053780 | A1 | 21 February 2019 | CN | 108697354 | A | 23 October 2018 |
| | | | | EP | 3419516 | A4 | 09 October 2019 |
| | | | | EP | 3419516 | A1 | 02 January 2019 |
| | | | | WO | 2017146886 | A1 | 31 August 2017 |
| US | 2018109698 | A1 | 19 April 2018 | CA | 3013926 | A1 | 17 August 2017 |
| | | | | WO | 2017139367 | A1 | 17 August 2017 |
| | | | | CN | 108882902 | A | 23 November 2018 |
| | | | | MX | 2018009566 | A | 30 May 2019 |
| | | | | KR | 20180115725 | A | 23 October 2018 |
| | | | | JP | 2019511342 | A | 25 April 2019 |
| | | | | EP | 3399916 | A4 | 07 August 2019 |
| | | | | EP | 3399916 | A1 | 14 November 2018 |

Form PCT/ISA/210 (patent family annex) (January 2015)